# EUROPEAN PATENT SPECIFICATION

(11) **EP 4 140 915 B1**
(45) Date of publication and mention of the grant of the patent: **24.12.2025**
(21) Application number: 21833591.7
(22) Date of filing: 30.03.2021
(51) Int. Cl.: B65D 77/20, A61M 5/172, A61B 5/01, A61B 5/25

(54) **STICK-ON TYPE BIOLOGICAL DEVICE COVER**
FESTSTECKBARE ABDECKUNG FÜR EINE BIOLOGISCHE VORRICHTUNG
COUVERCLE DE DISPOSITIF BIOLOGIQUE DU TYPE ADHÉSIF

(30) Priority: 03.07.2020 JP 2020115740
(43) Date of publication of application: 01.03.2023
(73) Proprietor: Murata Manufacturing Co., Ltd., Nagaokakyo-shi, Kyoto 617-8555 (JP)
(72) Inventor: SHIMUTA Toru, Nagaokakyo-shi, Kyoto 617-8555 (JP)
(74) Representative: Lorenz Seidler Gossel Part. mbB
(86) International application number: PCT/JP2021/013627
(87) International publication number: WO 2022/004079

(56) References cited:
- WO-A1-2014/189545
- WO-A1-97/42903
- WO-A1-99/55410
- WO-A2-2015/055289
- JP-A- 2004 147 855
- JP-A- 2016 505 808
- JP-A- 2018 075 256
- US-A- 4 704 177
- US-A1- 2008 076 987

## Description

### Technical Field

The present invention relates to a patch-type biomedical device cover that encloses a patch-type biomedical device, such as a biomedical sensor, to be adhered to a human body when in use.

### Background Art

An patch-type biomedical sensor (a biomedical device) is known. The patch-type biomedical sensor is adhered to a human body when in use to acquire biomedical information including, for example, body temperature and electrocardiography signals. When such a patch-type biomedical sensor is used, for example, in a hospital, the biomedical sensor is adhered to a human body using a double-sided adhesive sheet or the like, and a dressing film or a surgical tape is adhered thereon, thereby preventing the biomedical sensor from being stained with an antiseptic solution or the like (for example, see JP H01-201128 A).

Reference WO 2014/189545 A1 discloses a patch-type biomedical sensor of the generic kind. Further prior art includes WO 97/42903 A1, WO 99/55410 A1, US 2008/076987 A1, WO 2015/055289 A2, and US 4 704 177 A.

### Summary of Invention

### Technical Problem

When the above-described patch-type biomedical sensor is used repeatedly (reused), it is typical to do as follows. When in use, a double-sided adhesive sheet or the like is adhered to the bottom surface of the biomedical sensor, and the biomedical sensor is adhered to a human body (skin). Subsequently, a dressing film being cut into an appropriate size is adhered over the biomedical sensor. After use, the dressing film is peeled off and thrown away, and the biomedical sensor is recovered after the double-sided adhesive sheet is peeled off from the biomedical sensor.

It takes some time and efforts to attach and detach the biomedical sensor and the dressing film or the like, which is not convenient for use. In the case of the biomedical sensor using a relatively soft exterior material, such as urethane, the exterior material may be damaged when the dressing film or the like is peeled off.

The present invention is made to solve the above problem, and an object of the present invention is to provide a patch-type biomedical device cover for enclosing a patch-type biomedical device, such as a biomedical sensor, to be adhered to a human body when in use, the patch-type biomedical device cover being easily replaceable while preventing the patch-type biomedical device (especially an exterior portion) from being deteriorated or damaged during the replacement. The invention is defined by the appended claims 1-10.

### Solution to Problem

The present invention provides a patch-type biomedical device cover according to claim 1. The patch-type biomedical device cover includes a cover film, a first adhesive member, and a second adhesive member. The cover film includes an upper cover film and a lower cover film and is configured to be folded into a bag when in use so as to be able to accommodate a biomedical device. The first adhesive member has adhesiveness and is disposed on an outside surface of the lower cover film. The second adhesive member has adhesiveness and is disposed on an inside surface of the lower cover film. The second adhesive member is configured to adhere the biomedical device to the lower cover film and to adhere an edge portion of the upper cover film to an edge portion of the lower cover film so as to close the upper cover film and the lower cover film. The upper cover film has an area larger than that of the lower cover film.

According to the patch-type biomedical device cover of the present invention, the cover film includes the upper cover film and the lower cover film that are formed into a bag when in use so as to accommodate the biomedical device. This enables a user to easily put the biomedical device into, and take it out of, the cover film (i.e., the gap between the upper cover film and the lower cover film). The patch-type biomedical device cover also include the second adhesive member that is configured to adhere the biomedical device to the lower cover film and to adhere the edge portion of the upper cover film to the edge portion of the lower cover film. This enables the user to adhere the biomedical device easily to the inside surface of the lower cover film (inside the cover film) and the user to close the cover film easily. In addition, the first adhesive member having adhesiveness is disposed on the outside surface of the lower cover film, which enables the user to adhere the biomedical device to a human body easily with the cover film (the lower cover film) being interposed therebetween. Advantageous Effects of Invention

According to the present invention, the patch-type biomedical device cover encloses a patch-type biomedical device, such as a biomedical sensor, to be adhered to a human body when in use. The patch-type biomedical device cover enables a user to replace the patch-type biomedical device easily while reducing the likelihood of the patch-type biomedical device (especially the exterior portion) being deteriorated or damaged during the replacement.

### Brief Description of Drawings

[Fig. 1] Fig. 1 is a plan view illustrating a patch-type biomedical device cover (before use) according to an embodiment.
[Fig. 2] Fig. 2 is a side view illustrating the patch-type biomedical device cover (before use) according to the embodiment.
[Fig. 3] Fig. 3 is a cross-sectional view illustrating the patch-type biomedical device cover (when in use) according to the embodiment.
[Fig. 4] Fig. 4 is a cross-sectional view illustrating a structure of a patch-type temperature sensor.
[Fig. 5] Fig. 5 is a side view illustrating a patch-type biomedical device cover (before use) according to a first modification.
[Fig. 6] Fig. 6 includes side and plan views illustrating the patch-type biomedical device cover (when a temperature sensor is adhered thereto) according to the first modification.
[Fig. 7] Fig. 7 is a cross-sectional view illustrating the patch-type biomedical device cover (when in use) according to the first modification.
[Fig. 8] Fig. 8 is a side view illustrating a patch-type biomedical device cover (before use) according to a second modification (not a part of the claimed invention).
[Fig. 9] Fig. 9 includes side and plan views illustrating the patch-type biomedical device cover (when a temperature sensor is adhered thereto) according to the second modification.
[Fig. 10] Fig. 10 is a cross-sectional view illustrating the patch-type biomedical device cover (when in use) according to the second modification.
[Fig. 11] Fig. 11 is a plan view illustrating a patch-type biomedical device cover (before use) according to a third modification. Description of Embodiment

A preferred embodiment of the present invention will be described in detail with reference to the drawings. Note that the same or equivalent elements in the drawings are denoted by the same reference signs. In reference to each drawing, duplicated descriptions on the same elements denoted by the same reference signs are omitted.

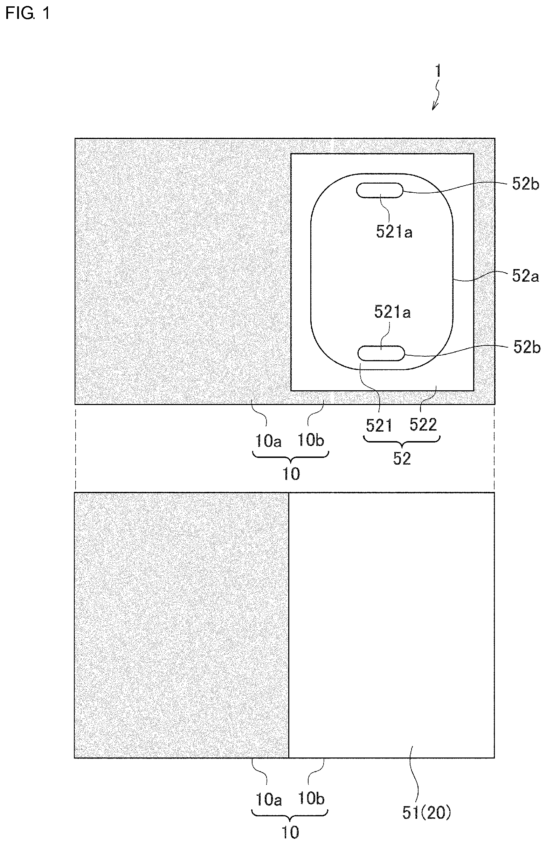

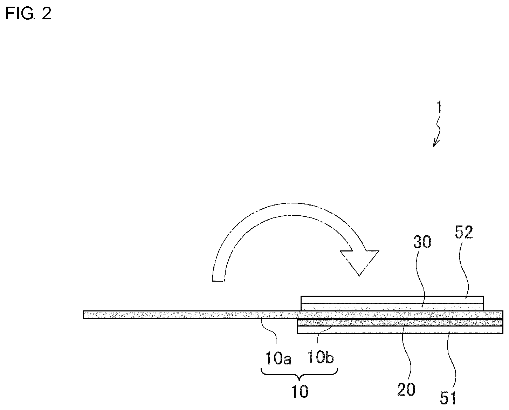

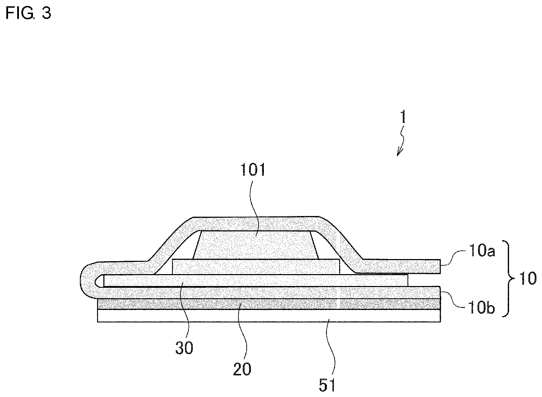

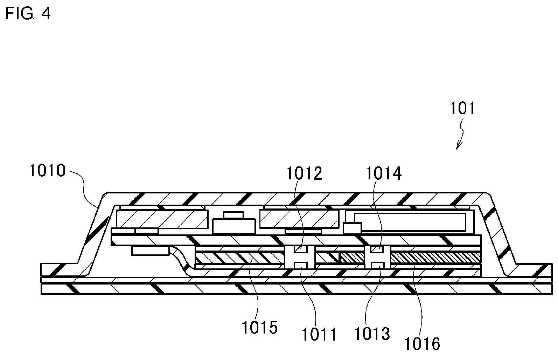

First, a structure of a patch-type biomedical device cover 1 according to an embodiment will be described with reference to Figs. 1 to 4. Note that the present invention will be described by taking an example in which the present invention is applied to a patch-type temperature sensor (deep body thermometer) 101 that is adhered to a human body to measure deep body temperature of the body. The patch-type temperature sensor corresponds to the biomedical device defined by the claims Fig. 1 is a plan view illustrating the patch-type biomedical device cover 1 before use (i.e., in a state at factory, on market, or in storage, etc.). Fig. 2 is a side view illustrating the patch-type biomedical device cover 1 before use. Fig. 3 is a cross-sectional view illustrating the patch-type biomedical device cover 1 when in use. Fig. 4 is a cross-sectional view illustrating a structure of the patch-type temperature sensor 101.

In the present embodiment, the patch-type temperature sensor 101 is a dual-heat-flux-type deep body thermometer equipped with a pair of thermal resistors 1015 and 1016 and two pairs of thermistors 1011, 1012, 1013, and 1014. An example of the dual-heat-flux-type deep body thermometer is described in International Publication No. WO 2019/225532, which can be preferably used as the patch-type temperature sensor 101.

The patch-type biomedical device cover 1 is a cover for enclosing the temperature sensor 101 to be adhered to a body when in use. The patch-type biomedical device cover 1 is a disposable cover. The patch-type biomedical device cover 1 has a function of easy replacement while reducing the likelihood of the patch-type temperature sensor 101 (especially an exterior portion 1010) being deteriorated or damaged during the replacement of the sensor cover 1.

For this purpose, the patch-type biomedical device cover 1 includes, as main elements, a cover film 10 composed of an upper cover film 10a and a lower cover film 10b, a first adhesive member 20, and a second adhesive member 30.

The cover film 10 having the upper cover film 10a and the lower cover film 10b can be formed into a bag that can accommodate the temperature sensor 101 when in use. In the present embodiment, the upper cover film 10a and the lower cover film 10b have respective rectangular shapes, and only one side of the upper cover film 10a is joined to one side of the lower cover film 10b. In other words, the upper cover film 10a and the lower cover film 10b are joined to each other at the one side so as to be foldable or bendable along the one side that extends linearly.

The upper cover film 10a and the lower cover film 10b are joined together (not separated), which can reduce the likelihood of the upper cover film 10a or the lower cover film 10b being lost. The upper cover film 10a can be opened completely by 180 degrees relative to the lower cover film 10b because the upper cover film 10a and the lower cover film 10b are joined only at one side, which makes it easy to peel a second separator 52 (to be described later) from the second adhesive member 30 and to adhere the temperature sensor 101 thereto. This also makes it easy to adhere the temperature sensor 101 at a desired position (predetermined position).

The upper cover film 10a is formed so as to have a larger area than that of the lower cover film 10b. For example, in the present embodiment, the length of the upper cover film 10a is made greater than the length of the lower cover film 10b in the folding direction of the upper cover film 10a (in other words, in the lateral direction in each drawing). Accordingly, the area of the upper cover film 10a is made larger than the area of the lower cover film 10b. Since the temperature sensor 101 to be accommodated in the cover film 10 has a certain thickness, the larger area of the upper cover film 10a larger reduces the likelihood of the upper cover film 10a not covering the second adhesive member 30 and the likelihood of the second adhesive member 30 being exposed when the upper cover film 10a and the lower cover film 10b are folded into a bag.

The cover film 10 (i.e., the upper cover film 10a and the lower cover film 10b) is preferably made of a resin film, which is flexible and resistant to breakage. It is especially preferable to use polyethylene terephthalate (PET) or polypropylene (PP), which are colorless and transparent and are not drawn easily. When the cover is transparent, the temperature sensor 101 under the upper cover film 10a can be observed therethrough, which is helpful to notice mispositioning of the temperature sensor 101. In the case of using PET, the thickness of the cover is preferably in the range of 12 to 100 µm, and more preferably 25 to 50 µm. In the case of using PP (for example, bi-axially oriented polypropylene (BOPP) or cast polypropylene (CPP)), the thickness is preferably in the range of 12 to 150 µm, and more preferably 30 to 70 µm. For example, polyethylene (PE), polyimide (PI), nylon, or polyvinyl chloride (PVC) can be used in place of PET or PP.

The first adhesive member 20 having adhesiveness is disposed on (adhered to) the outside surface of the lower cover film 10b. The first adhesive member 20 is provided to adhere the patch-type biomedical device cover 1 to a human body with the temperature sensor 101 being enclosed therein. It is preferable that the first adhesive member 20 be a rectangular sheet and cover the entire outside surface of the lower cover film 10b, in other words, it is preferably that the first adhesive member 20 have the same dimensions as the lower cover film 10b. If the lower cover film 10b has a non-adhesive region (in other words, a region not covered by the first adhesive member 20) between the lower cover film 10b and a human body (an object to be adhered to), the lower cover film 10b peels off easily from the non-adhesive region. In the present embodiment, however, the first adhesive member 20 covers the entire surface of the lower cover film 10b, which reduces the likelihood of the cover film 10 (patch-type biomedical device cover 1) peeling off easily.

The first adhesive member 20 may preferably be a double-sided adhesive member for body use, which is made of, for example, an acryl-based adhesive or a silicone-based adhesive. The adhesion strength of the first adhesive member 20 adhering to the cover film 10 is preferably in the range of 5 to 20 (N/20mm), and more preferably 10 to 15 (N/20mm). The adhesion strength of the first adhesive member 20 adhering to skin (object to be adhered to) is preferably in the range of 0.5 to 15 (N/20mm), and more preferably 5 to 10 (N/20mm). The thickness of the first adhesive member 20 is preferably in the range of 5 to 400 µm, and more preferably 100 to 200 µm. A resin film (PET, PE, or the like) may be used as a substrate for the first adhesive member 20. A non-woven fabric substrate or a paper substrate may be used in place of the resin film substrate. The first adhesive member 20 does not necessarily include the substrate.

The second adhesive member 30 having adhesiveness is disposed on (adhered to) the inside surface of the lower cover film 10b. The second adhesive member 30 is provided to adhere the patch-type temperature sensor 101 to the lower cover film 10b and to adhere the edge portion of the upper cover film 10a to the edge portion of the lower cover film 10b (in other words, to form the cover film 10 into a bag). The second adhesive member 30 is disposed so as to oppose the first adhesive member 20 with the lower cover film 10b being interposed therebetween.

For example, the second adhesive member 30 is a rectangular sheet. As viewed in plan (i.e., as viewed in the thickness direction), the outer shape of the second adhesive member 30 (i.e., the area of the second adhesive member 30) is preferably made larger than the outer shape of the temperature sensor 101 (i.e., the area of the temperature sensor 101) and smaller than the outer shape of the lower cover film 10b (i.e., the area of the lower cover film 10b). If the second adhesive member 30 is present to the edge of the cover film 10, a user cannot pull apart the edge portion of the cover film 10 (i.e., edge portions of the upper cover film 10a and the lower cover film 10b). With the above configuration, however, the user can open the cover film 10 easily after use by pulling apart the edge portions of the upper cover film 10a and the lower cover film 10b.

The second adhesive member 30 includes a first adhesion region to which the temperature sensor 101 is adhered and a second adhesion region to which the edge portion of the upper cover film 10a is adhered, details of which will be described later. With this configuration, the user can adhere the temperature sensor 101 to the lower cover film 10b easily and can cover the temperature sensor 101 with the upper cover film 10a easily.

The adhesion strength of the second adhesive member 30 adhering to the cover film 10 is preferably set to be smaller than the adhesion strength of the first adhesive member 20 adhering to the cover film 10. The strength of adhesion can be adjusted, for example, by changing the adhesion area of each layer or by changing the adhesive material to be used.

This makes it easier to adjust the position of the temperature sensor 101 and adhere it again, for example, after the temperature sensor 101 is attached and the upper cover film 10a is closed. If the second adhesive member 30 had a greater adhesion strength, the first adhesive member 20 would come off first from the lower cover film 10b when the user tries to peel the upper cover film 10a off after the cover film 10 is adhered, for example, to a human body (skin). Note that it is not a problem that the first adhesive member 20 peels off first from the human body (object to be adhered to), which is rather convenient in the case of redoing the cover film 10. However, if the first adhesive member 20 is detached from the lower cover film 10b, the first adhesive member 20 may become wrinkled and cannot be adhered again to the lower cover film 10b.

For example, the second adhesive member 30 may preferably be a double-sided adhesive member, which is made of an acryl-based adhesive. The adhesion strength of the second adhesive member 30 adhering to the cover film 10 is preferably in the range of 0.2 to 15 (N/20mm), and more preferably 5 to 10 (N/20mm). The adhesion strength of the second adhesive member 30 adhering to the temperature sensor 101 is preferably in the range of 0.2 to 15 (N/20mm), and more preferably 1 to 10 (N/20mm). The thickness of the second adhesive member 30 is preferably in the range of 5 to 300 µm, and more preferably 10 to 100 µm. A resin film (PET, PE, or the like) may be used as a substrate for the second adhesive member 30. A non-woven fabric substrate or a paper substrate may be used in place of the resin film substrate. The second adhesive member 30 does not necessarily include the substrate.

Thus, when the cover film 10 is used, the cover film 10 is configured such that the upper cover film 10a, the temperature sensor 101, the second adhesive member 30, the lower cover film 10b, and the first adhesive member 20 are layered.

Next, the configuration of the patch-type biomedical device cover 1 before use (i.e., in a state at factory, on market, or in storage, etc.) will be described.

Before use, the patch-type biomedical device cover 1 has a first separator 51 and a second separator 52. The first separator 51 is releasably adhered to the above-described first adhesive member 20 so as to cover the first adhesive member 20. The second separator 52 is releasably adhered to the second adhesive member 30 so as to cover the second adhesive member 30.

In other words, the first separator 51 and the second separator 52 are members that are adhered to adhesive surfaces of the patch-type biomedical device cover 1 (i.e., surfaces of the first adhesive member 20 and the second adhesive member 30) when the patch-type biomedical device cover 1 is not in use. The first separator 51 and the second separator 52 are peeled off from the adhesive surfaces of the patch-type biomedical device cover 1 (i.e., surfaces of the first adhesive member 20 and the second adhesive member 30) when in use.

For example, the first separator 51 may preferably be a resin film (PET) or a sheet of paper finished with releasing treatment. The thickness of the first separator 51 is preferably in the range of 50 to 500 µm, and more preferably 100 to 400 µm. The first separator 51 is preferably made of a relatively rigid material (paper or PET) and be made relatively thick in order to increase the rigidity. The first separator 51 is preferably not transparent. This enables the user to easily recognize that it is necessary to peel the first separator 51 off (if it is transparent, the user does not notice it easily).

For example, the second separator 52 may preferably be a resin film (PET) or a sheet of paper finished with releasing treatment. The thickness of the second separator 52 is preferably in the range of 20 to 500 µm, and more preferably 50 to 200 µm. The second separator 52 is preferably not transparent. This enables the user to easily recognize that it is necessary to peel the second separator 52 off (if it is transparent, the user does not notice it easily). Moreover, the user can easily notice instructions or illustrations printed on the non-transparent second separator 52, which enables the user to immediately understand how to use.

A slit 52a is formed in the second separator 52. The slit 52a divides the second separator 52 into a first separator portion 521 and a second separator portion 522. The first separator portion 521 covers the first adhesion region of the second adhesive member 30 to which the temperature sensor 101 is adhered. The second separator portion 522 covers the second adhesion region of the second adhesive member 30 to which the edge portion of the upper cover film 10a is adhered.

For example, the first separator portion 521 (i.e., the first adhesion region) is shaped like a rectangle with corners being rounded off so as to be similar to, but larger than, the bottom shape of the temperature sensor 101. On the other hand, the second separator portion 522 (second adhesion region) is shaped annularly so as to surround the first adhesion region. The slit 52a, however, does not need to have a closed-loop like shape. In other words, the slit 52a may be in contact with an edge of the second separator 52. For example, the second separator 52 may be divided equally by a straight line, and the second separator 52 may be peeled off half by half when in use.

With the above configuration, the user can peel off only the separator member that covers the first adhesion region to which the temperature sensor 101 is adhered (in other words, peel off only the first separator portion 521), which enables the user to adhere the temperature sensor 101 to the designated position accurately. This can prevent a hand or clothes from touching the region (second adhesion region) to which the edge portion of the upper cover film 10a is subsequently adhered and thereby prevent the adhesion strength of the second adhesion region from deteriorating.

The first separator portion 521 of the second separator 52 preferably includes an island region (small cutout portion) 521a defined by a closed-loop slit 52b that is not in contact with the periphery (peripheral edge) of the first separator portion 521. When the first separator portion 521 is peeled off, part of the first separator portion 521 remains as the island region 521a on the second adhesive member 30. It is preferable to provide multiple island regions (small cutout portions) 521a. In the present embodiment, two island regions 521a are provided. The temperature sensor 101 does not adhere to the second adhesive member 30 in the island regions (small cutout portions) 521a, which enables the user to detach the temperature sensor 101 easily from the second adhesive member 30.

The first adhesive member 20 and the first separator 51 have respective sides near the joining portion between the upper cover film 10a and the lower cover film 10b. It is preferable that the sides of the first adhesive member 20 and the lower cover film 10b extend linearly (in other words, includes a linearly extending portion) in a direction substantially orthogonal to the folding (bending) direction of the upper cover film 10a. This enables the user to fold the upper cover film 10a neatly along the linear portion extending in the direction orthogonal to the folding direction of the first separator 51.

It is preferable that the first separator 51 and/or the first adhesive member 20 have a greater rigidity compared with the upper cover film 10a, the lower cover film 10b, and the second adhesive member 30. This enables the user to fold the upper cover film 10a easily along the edge of the first separator 51 and the first adhesive member 20. Moreover, the lower cover film 10b does not bend easily, which reduces the likelihood of bubbles being formed between the temperature sensor 101 and the second adhesive member 30 when the temperature sensor 101 is adhered to the lower cover film 10b.

When a user uses the patch-type temperature sensor 101, the user first peels off the first separator portion 521 of the second separator 52 and adheres the temperature sensor 101 to the first adhesion region of the second adhesive member 30. Subsequently, the user peels off the second separator portion 522 of the second separator 52, folds (bends) the upper cover film 10a toward the lower cover film 10b, and adheres the edge portion of the upper cover film 10a to the edge portion of the lower cover film 10b. Next, the user peels the first separator 51 off and adheres the cover film 10 (patch-type biomedical device cover 1) enclosing the temperature sensor 101 to a human body (skin).

After the temperature (deep body temperature) of the body is measured, in other words, after the patch-type temperature sensor 101 is used, the user detaches the cover film 10 (patch-type biomedical device cover 1) enclosing the temperature sensor 101 from the body (skin). Next, the user opens the cover film 10, detaches the temperature sensor 101 from the second adhesive member 30. Thus, the temperature sensor 101 is recovered. The patch-type biomedical device cover 1 is thrown away after use.

According to the present embodiment, as described above in detail, the cover film 10 has the upper cover film 10a and the lower cover film 10b and is formed into a bag that can accommodate the temperature sensor 101 when in use. Thus, the temperature sensor 101 can be easily put into, and taken out of, the cover film 10 (i.e., into and out of the gap between the upper cover film 10a and the lower cover film 10b). In addition, the second adhesive member 30 is disposed on the inside surface of the lower cover film 10b to adhere the temperature sensor 101 to the lower cover film 10b and to adhere the edge portion of the upper cover film 10a to the edge portion of the lower cover film 10b. This enables the user to adhere the temperature sensor 101 easily to the inside surface of the lower cover film 10b (inside the cover film 10) and also to close the cover film 10 easily. Moreover, the first adhesive member 20 having adhesiveness is disposed on the outside surface of the lower cover film 10b, which enables the user to adhere the temperature sensor 101 easily to a human body with the cover film 10 (lower cover film 10b) being interposed therebetween.

Thus, according to the present embodiment, the temperature sensor 101 can be replaced easily while reducing the likelihood of the temperature sensor 101 (especially the exterior portion 1010) being deteriorated or damaged or the like during the replacement. Even when it is difficult to use heating or an antiseptic solution, etc., to sterilize the temperature sensor 101 (biomedical device), the replacement of the above-described cover can meet the hygiene requirements.

### First Modification

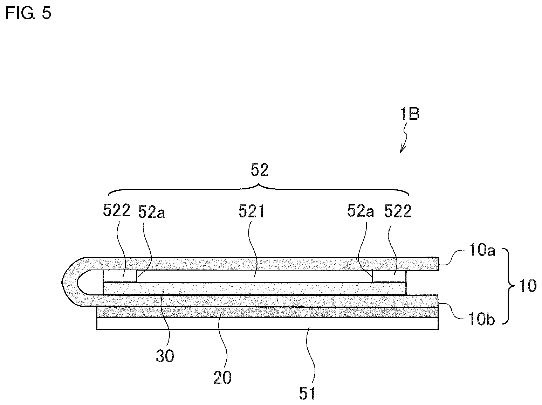

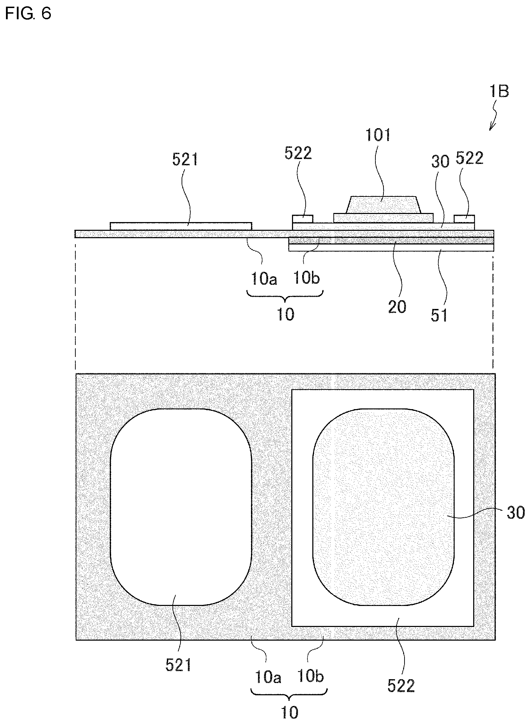

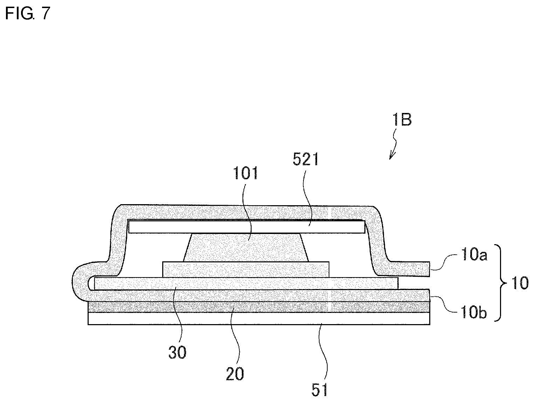

Next, a patch-type biomedical device cover 1B according to a first modification will be described with reference to Figs. 5 to 7. Fig. 5 is a side view illustrating the patch-type biomedical device cover 1B before use (i.e., in a state at factory, on market, or in storage, etc.). Fig. 6 includes side and plan views illustrating the patch-type biomedical device cover 1B to which the temperature sensor 101 is adhered. Fig. 7 is a cross-sectional view illustrating the patch-type biomedical device cover 1B when in use.

The patch-type biomedical device cover 1B is different from the above-described patch-type biomedical device cover 1 in that the inside surface of the upper cover film 10a is adhered (fixed) to the first separator portion 521 of the second separator 52. For example, an adhesive or a double-sided adhesive tape can be used to adhere (fix) the upper cover film 10a to the first separator portion 521 of the second separator 52.

According to the present modification, when a user peels the first separator portion 521 or the second separator portion 522 of the second separator 52, the patch-type biomedical device cover 1B can reduce the likelihood of the user peeling off a wrong separator portion first. More specifically, when the user opens the upper cover film 10a when in use, the upper cover film 10a peels the first separator portion 521 from the second adhesive member 30. Accordingly, the user can immediately recognize that it is the remaining second separator portion 522 for the user to peel off next. Moreover, when the user adheres the upper cover film 10a to the lower cover film 10b, the user visually aligns the second separator portion 522 fixed on the upper cover film 10a with respect to the temperature sensor 101, which enables the user to adhere the upper cover film 10a correctly.

Note that other configurations are the same as or similar to those of the patch-type biomedical device cover 1 described above, and detailed descriptions thereof are not repeated here.

### Second Modification (not a part of the claimed invention)

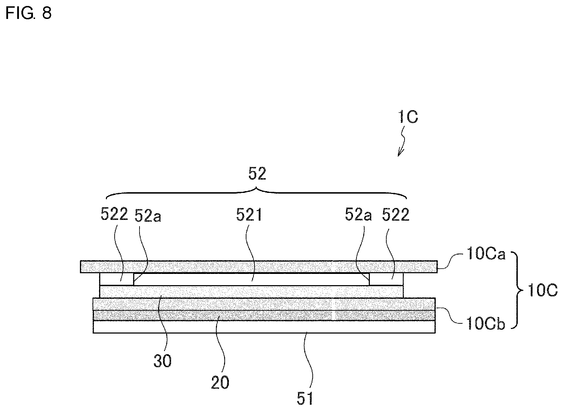

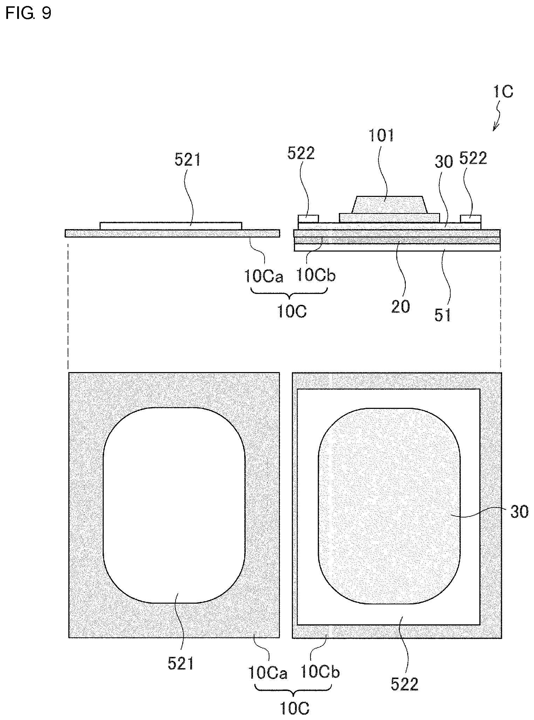

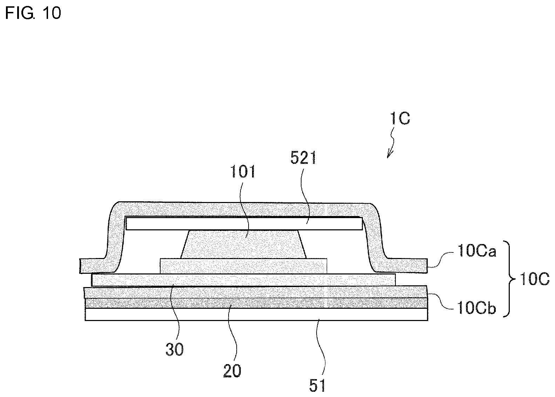

In the above embodiment, the upper cover film 10a and the lower cover film 10b are joined at the one side. The upper cover film 10a and the lower cover film 10b, however, may be separated from each other as illustrated in Figs. 8 to 10. A patch-type biomedical device cover 1C according to a second modification will be described with reference to Figs. 8 to 10. Fig. 8 is a side view of the patch-type biomedical device cover 1C before use (i.e., in a state at factory, on market, or in storage, etc.). Fig. 9 includes side and plan views of the patch-type biomedical device cover 1C to which the temperature sensor 101 is adhered. Fig. 10 is a cross-sectional view of the patch-type biomedical device cover 1C when in use.

The patch-type biomedical device cover 1C is different from the above-described patch-type biomedical device cover 1 or 1B in that an upper cover film 10Ca and a lower cover film 10Cb included in a cover film 10C are separated from each other (i.e., not connected to each other).

According to the present modification, as is the case for the patch-type biomedical device cover 1, the temperature sensor 101 can be replaced easily while reducing the likelihood of the temperature sensor 101 (especially the exterior portion 1010) being deteriorated or damaged during the replacement.

Note that other configurations are the same as or similar to those of the above-described patch-type biomedical device cover 1 or 1B, and detailed descriptions thereof are not repeated here.

### Third Modification

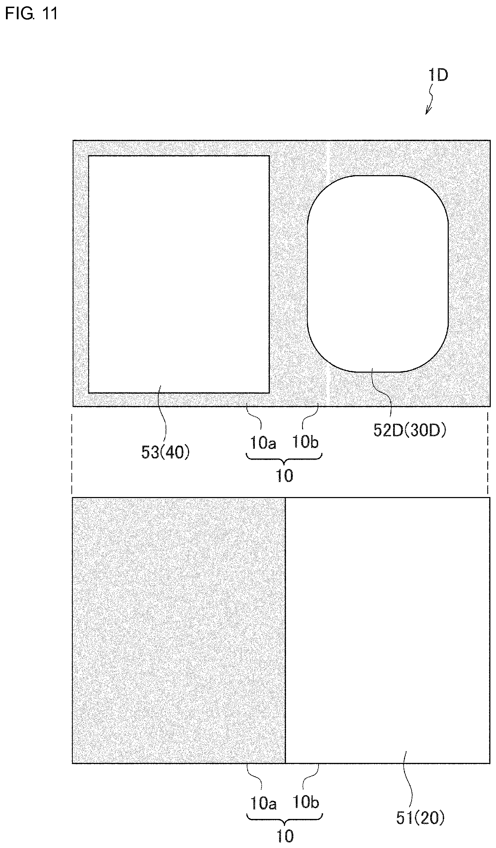

Next, a patch-type biomedical device cover 1D according to a third modification will be described with reference to Fig. 11. Fig. 11 is a plan view illustrating the patch-type biomedical device cover 1D before use (i.e., in a state at factory, on market, or in storage, etc.).

The patch-type biomedical device cover 1D is different from the above-described patch-type biomedical device cover 1 or 1B in that the patch-type biomedical device cover 1D further includes a third adhesive member 40 that has adhesiveness and is disposed on the inside surface of the upper cover film 10a and in that the patch-type biomedical device cover 1D includes a third separator 53 that is releasably adhered to the third adhesive member 40.

In addition, the patch-type biomedical device cover 1D is different from the above-described patch-type biomedical device cover 1 or 1B in that the patch-type biomedical device cover 1D includes a second adhesive member 30D in place of the second adhesive member 30 (more specifically, the second adhesive member 30D includes only the first adhesion region of the above-described second adhesive member 30). Note that the third adhesive member 40 may be connected to the second adhesive member 30D.

Moreover, the patch-type biomedical device cover 1D is different from the above-described patch-type biomedical device cover 1 or 1B in that the patch-type biomedical device cover 1D includes a second separator 52D in place of the second separator 52 (more specifically, the second separator 52D includes only the first separator portion 521 of the second separator 52).

In this case, the user peels the third separator 53 off when in use and adheres the third adhesive member 40 to the top surface of the temperature sensor 101 and to the edge portion of the lower cover film 10b. Note that the adhesion strength of the third adhesive member 40 is preferably small in order to reduce the likelihood of the top surface of the temperature sensor 101 being damaged or the like when the third adhesive member 40 is peeled off after use.

According to the present modification, even if the upper cover film 10a is mispositioned and the adhesion area between the upper cover film 10a and the lower cover film 10b becomes small, the upper cover film 10a is still adhered to the top surface of the temperature sensor 101, which can reduce the likelihood of the upper cover film 10a peeling off.

Note that other configurations are the same as or similar to those of the above-described patch-type biomedical device cover 1 or 1B, and detailed descriptions thereof are not repeated here.

The present invention has been described using an example in which the present invention is applied to the temperature sensor 101 of a patch-type. The present invention, however, can be applied, for example, to patch-type electrocardiography sensors, acceleration sensors, or other biomedical devices.

### Reference Signs List

1, 1B, 1C, 1D patch-type biomedical device cover
10, 10C cover film
10a, 10Ca upper cover film
10b, 10Cb lower cover film
20 first adhesive member
30, 30D second adhesive member
40 third adhesive member
51 first separator
52, 52D second separator
52a, 52b slit
521 first separator portion
521a island region (small cutout portion)
522 second separator portion
53 third separator
101 patch-type temperature sensor (biomedical device)

## Claims

1. A patch-type biomedical device cover (1B), comprising:
a cover film (10) that includes an upper cover film (10a) and a lower cover film (10b) and is configured to be folded into a bag when in use so as to be able to accommodate a biomedical device (101);
a first adhesive member (20) that has adhesiveness and is disposed on an outside surface of the lower cover film (10b); and
a second adhesive member (30) that has adhesiveness and is disposed on an inside surface of the lower cover film (10b),
wherein the second adhesive member (30) is configured to adhere the biomedical device (101) to the lower cover film (10b) and to adhere an edge portion of the upper cover film (10a) to an edge portion of the lower cover film (10b) so as to close the upper cover film (10a) and the lower cover film (10b); **characterized in that**
the upper cover film (10a) has an area larger than that of the lower cover film (10b).

2. The patch-type biomedical device cover (1B) according to Claim 1, wherein the first adhesive member (20) is disposed so as to entirely cover the outside surface of the lower cover film (10b).

3. The patch-type biomedical device cover (1B) according to any one of Claims 1 to 2, wherein
as viewed in plan, an outer shape of the second adhesive member (30) is larger than an outer shape of the biomedical device (101) and is smaller than an outer shape of the lower cover film (10b).

4. The patch-type biomedical device cover (1B) according to any one of Claims 1 to 3, wherein
an adhesion strength of the second adhesive member (30) is smaller than an adhesion strength of the first adhesive member (20).

5. The patch-type biomedical device cover according to any one of Claims 1 to 4, further comprising:
a first separator (51) that is releasably adhered to the first adhesive member (20); and
a second separator (52) that is releasably adhered to the second adhesive member (30), wherein
a slit (52a) is formed in the second separator (52),
the slit (52a) divides the second separator (52) into a first separator portion (521) and a second separator portion (522),
the first separator portion (521) covers a first adhesion region of the second adhesive member (30) to which the biomedical device (101) is adhered, and
the second separator portion (522) covers a second adhesion region of the second adhesive member (30) to which the edge portion of the upper cover film (10a) is adhered.

6. The patch-type biomedical device cover (1B) according to Claim 5, wherein
the first separator portion (521) of the second separator (52) includes a region that is defined by a closed-loop slit that is not in contact with a periphery of the first separator portion (521), and the region remains on the second adhesive member (30) when the first separator portion (521) is peeled off.

7. The patch-type biomedical device cover (1B) according to Claim 5 or 6, wherein
an inside surface of the upper cover film (10a) is adhered to the first separator portion (521) of the second separator (52).

8. The patch-type biomedical device cover (1B) according to any one of the preceding Claims, wherein
the upper cover film (10a) and the lower cover film (10b) are joined to each other along a line extending linearly and are foldable along the line.

9. The patch-type biomedical device cover (1B) according to Claim 8, wherein
the first adhesive member (20) and the first separator (51) have respective sides at a joining portion between the upper cover film (10a) and the lower cover film (10b), and the sides are formed so as to extend linearly in a direction substantially orthogonal to a folding direction of the upper cover film (10a).

10. The patch-type biomedical device cover (1B) according to Claim 8 or 9, wherein
the first separator (51) and/or the first adhesive member (20) have a greater rigidity compared with the upper cover film (10a), the lower cover film (10b), and the second adhesive member (30).

## Patentansprüche

1. Pflasterartige Abdeckung (1B) für eine biomedizinische Vorrichtung, umfassend:
eine Abdeckfolie (10), die eine obere Abdeckfolie (10a) und eine untere Abdeckfolie (10b) umfasst und dazu ausgebildet ist, im Gebrauch zu einem Beutel gefaltet zu werden, um eine biomedizinische Vorrichtung (101) aufnehmen zu können;
ein erstes Klebeelement (20), das Klebrigkeit besitzt und auf einer Außenseite der unteren Abdeckfolie (10b) angeordnet ist; und
ein zweites Klebeelement (30), das Klebrigkeit besitzt und auf einer Innenseite der unteren Abdeckfolie (10b) angeordnet ist,
wobei das zweite Klebeelement (30) dazu ausgebildet ist, die biomedizinische Vorrichtung (101) auf die untere Abdeckfolie (10b) zu kleben und einen Randabschnitt der oberen Abdeckfolie (10a) auf einen Randabschnitt der unteren Abdeckfolie (10b) zu kleben, um die obere Abdeckfolie (10a) und die untere Abdeckfolie (10b) zu schließen;
**dadurch gekennzeichnet, dass**
die obere Abdeckfolie (10a) eine Fläche hat, die größer ist als die der unteren Abdeckfolie (10b).

2. Pflasterartige Abdeckung (1B) für eine biomedizinische Vorrichtung nach Anspruch 1, wobei
das erste Klebeelement (20) so angeordnet ist, dass es die Außenseite der unteren Abdeckfolie (10b) vollständig bedeckt.

3. Pflasterartige Abdeckung (1B) für eine biomedizinische Vorrichtung nach einem der Ansprüche 1 bis 2, wobei
in Draufsicht betrachtet, eine äußere Form des zweiten Klebeelements (30) größer ist als eine äußere Form der biomedizinischen Vorrichtung (101) und kleiner ist als eine äußere Form der unteren Abdeckfolie (10b).

4. Pflasterartige Abdeckung (1B) für eine biomedizinische Vorrichtung nach einem der Ansprüche 1 bis 3, wobei
eine Haftfestigkeit des zweiten Klebeelements (30) geringer ist als eine Haftfestigkeit des ersten Klebeelements (20).

5. Pflasterartige Abdeckung für eine biomedizinische Vorrichtung nach einem der Ansprüche 1 bis 4, ferner umfassend:
ein erstes Trennelement (51), das lösbar auf das erste Klebeelement (20) geklebt ist; und
ein zweites Trennelement (52), das lösbar auf das zweite Klebeelement (30) geklebt ist, wobei
ein Schlitz (52a) in dem zweiten Trennelement (52) ausgebildet ist,
der Schlitz (52a) das zweite Trennelement (52) in einen ersten Trennelementabschnitt (521) und einen zweiten Trennelementabschnitt (522) unterteilt,
der erste Trennelementabschnitt (521) einen ersten Klebebereich des zweiten Klebeelements (30) bedeckt, auf das die biomedizinische Vorrichtung (101) geklebt ist, und
der zweite Trennelementabschnitt (522) einen zweiten Klebebereich des zweiten Klebeelements (30) bedeckt, auf das der Randabschnitt der oberen Abdeckfolie (10a) geklebt ist.

6. Pflasterartige Abdeckung (1B) für eine biomedizinische Vorrichtung nach Anspruch 5, wobei
der erste Trennelementabschnitt (521) des zweiten Trennelements (52) einen Bereich aufweist, der durch einen geschlossenen Schlitz definiert ist, der nicht mit einem Umfang des ersten Trennelementabschnitts (521) in Kontakt steht, und wobei der Bereich auf dem zweiten Klebeelement (30) bleibt, wenn der erste Trennelementabschnitt (521) abgezogen wird.

7. Pflasterartige Abdeckung (1B) für eine biomedizinische Vorrichtung nach Anspruch 5 oder 6, wobei
eine Innenseite der oberen Abdeckfolie (10a) auf den ersten Trennelementabschnitt (521) des zweiten Trennelements (52) geklebt ist.

8. Pflasterartige Abdeckung (1B) für eine biomedizinische Vorrichtung nach einem der vorhergehenden Ansprüche, wobei
die obere Abdeckfolie (10a) und die untere Abdeckfolie (10b) entlang einer linear verlaufenden Linie miteinander verbunden sind und entlang der Linie faltbar sind.

9. Pflasterartige Abdeckung (1B) für eine biomedizinische Vorrichtung nach Anspruch 8, wobei
das erste Klebeelement (20) und das erste Trennelement (51) jeweils Seiten an einem Verbindungsabschnitt zwischen der oberen Abdeckfolie (10a) und der unteren Abdeckfolie (10b) aufweisen, und die Seiten so ausgebildet sind, dass sie in einer Richtung im Wesentlichen orthogonal zu einer Faltrichtung der oberen Abdeckfolie (10a) linear verlaufen.

10. Pflasterartige Abdeckung (1B) für eine biomedizinische Vorrichtung nach Anspruch 8 oder 9, wobei
das erste Trennelement (51) und/oder das erste Klebeelement (20) im Vergleich zu der oberen Abdeckfolie (10a), der unteren Abdeckfolie (10b) und dem zweiten Klebeelement eine größere Steifigkeit besitzen.

## Revendications

1. Couvercle de dispositif biomédical de type patch (1B), comprenant :
un film de couverture (10) qui comprend un film de couverture supérieur (10a) et un film de couverture inférieur (10b) et qui est configuré pour être plié en un sachet lorsqu'il est utilisé afin de pouvoir accueillir un dispositif biomédical (101) ;
un premier élément adhésif (20) qui possède une adhésivité et qui est disposé sur une surface extérieure du film de couverture inférieur (10b) ; et
un deuxième élément adhésif (30) qui possède une adhésivité et qui est disposé sur une surface intérieure du film de couverture inférieur (10b),
dans lequel le deuxième élément adhésif (30) est configuré pour faire adhérer le dispositif biomédical (101) au film de couverture inférieur (10b) et pour faire adhérer une partie de bord du film de couverture supérieur (10a) à une partie de bord du film de couverture inférieur (10b) de manière à fermer le film de couverture supérieur (10a) et le film de couverture inférieur (10b) ;
**caractérisé en ce que**
le film de couverture supérieur (10a) présente une surface plus grande que celle du film de couverture inférieur (10b).

2. Couvercle de dispositif biomédical de type patch (1B) selon la revendication 1, dans lequel
le premier élément adhésif (20) est disposé de manière à recouvrir entièrement la surface extérieure du film de couverture inférieur (10b).

3. Couvercle de dispositif biomédical de type patch (1B) selon l'une quelconque des revendications 1 à 2, dans lequel
vu en plan, une forme extérieure du deuxième élément adhésif (30) est plus grande qu'une forme extérieure du dispositif biomédical (101) et plus petite qu'une forme extérieure du film de couverture inférieur (10b).

4. Couvercle de dispositif biomédical de type patch (1B) selon l'une quelconque des revendications 1 à 3, dans lequel
une force d'adhésion du deuxième élément adhésif (30) est inférieure à une force d'adhésion du premier élément adhésif (20).

5. Couvercle de dispositif biomédical de type patch selon l'une quelconque des revendications 1 à 4, comprenant en outre :
un premier séparateur (51) qui est collé de manière amovible au premier élément adhésif (20) ; et
un deuxième séparateur (52) qui est collé de manière amovible au deuxième élément adhésif (30), dans lequel
une fente (52a) est formée dans le deuxième séparateur (52),
la fente (52a) divise le deuxième séparateur (52) en une première partie de séparateur (521) et une deuxième partie de séparateur (522),
la première partie de séparateur (521) recouvre une première zone d'adhésion du deuxième élément adhésif (30) auquel le dispositif biomédical (101) est collé, et
la deuxième partie de séparateur (522) recouvre une deuxième zone d'adhésion du deuxième élément adhésif (30) auquel la partie de bord du film de couverture supérieur (10a) est collée.

6. Couvercle de dispositif biomédical de type patch (1B) selon la revendication 5, dans lequel
la première partie de séparateur (521) du deuxième séparateur (52) comprend une zone qui est définie par une fente en boucle fermée qui n'est pas en contact avec une périphérie de la première partie de séparateur (521), et la zone reste sur le deuxième élément adhésif (30) lorsque la première partie de séparateur (521) est décollée.

7. Couvercle de dispositif biomédical de type patch (1B) selon la revendication 5 ou 6, dans lequel
une surface intérieure du film de couverture supérieur (10a) est collée à la première partie de séparateur (521) du deuxième séparateur (52).

8. Couvercle de dispositif biomédical de type patch (1B) selon l'une quelconque des revendications précédentes, dans lequel
le film de couverture supérieur (10a) et le film de couverture inférieur (10b) sont reliés l'un à l'autre le long d'une ligne s'étendant linéairement et sont pliables le long de la ligne.

9. Couvercle de dispositif biomédical de type patch (1B) selon la revendication 8, dans lequel
le premier élément adhésif (20) et le premier séparateur (51) possèdent des côtés respectifs au niveau d'une partie de jonction entre le film de couverture supérieur (10a) et le film de couverture inférieur (10b), et les côtés sont formés de manière à s'étendre linéairement dans une direction sensiblement orthogonale à une direction de pliage du film de couverture supérieur (10a).

10. Couvercle de dispositif biomédical de type patch (1B) selon la revendication 8 ou 9, dans lequel
le premier séparateur (51) et/ou le premier élément adhésif (20) possèdent une plus grande rigidité en comparaison avec le film de couverture supérieur (10a), le film de couverture inférieur (10b) et le deuxième élément adhésif (30).
